Europäisches Patentamt

European Patent Office

Office européen des brevets

(19)

(11) Publication number: 0 336 760 A2

# (12) EUROPEAN PATENT APPLICATION

(21) Application number: 89303417.3

(22) Date of filing: 06.04.89

(51) Int. Cl.⁴: C 07 K 7/00
A 61 K 37/02

(30) Priority: 06.04.88 US 178133   08.11.88 US 268835
23.01.89 US 299408

(43) Date of publication of application:
11.10.89 Bulletin 89/41

(84) Designated Contracting States:
AT BE CH DE ES FR GB GR IT LI LU NL SE

(71) Applicant: COLLAGEN CORPORATION
2500 Faber Place
Palo Alto, California 94303 (US)

(72) Inventor: Bentz, Hanne
36125 Toulouse Street
Newark California 94560 (US)

Nathan, Ranga
6104 Robertson Avenue
Newark California 94560 (US)

Rosen, David M.
3655 Shadyhollow Court
San Jose California 95148 (US)

Dasch, James R.
3181 Morris Drive
Palo Alto California 94303 (US)

Seyedin, Saeid M.
20761 Lowena Court
Saratoga California 95070 (US)

(74) Representative: Goldin, Douglas Michael et al
J.A. KEMP & CO. 14, South Square Gray's Inn
London WC1R 5EU (GB)

The microorganism(s) has (have) been deposited with ATCC-HB under numbers 10099 and 10098.

(54) Bone-inducing protein.

(57) An osteogenically active protein, having the sequence
(H₂N)-Ala-Lys-Tyr-Asn-Lys-Ile-Lys-Ser-Arg-Gly-Ile-Lys-Ala-
Asn-Thr-Phe-Lys-Lys-Leu-His-Asn-Leu-Ser-Phe-Leu-Tyr-Leu-
Asp-His-Asn-Ala-Leu-Glu-Ser-Val-Pro-Leu-Asn-Leu-Pro-Glu-
Ser-Leu-Arg-Val-Ile-His-Leu-Gln-Phe-Asn-Asn-Ile-Thr-Ser-Ile-
Thr-As   p-Asp-Thr-Phe-Cys-Lys-Ala-Asn-Asp-Thr-Ser-Tyr-Ile-
Arg-Asp-Arg-Ile-Glu-Glu-Ile-Arg-Leu-Glu-Gly-Asn-Pro-Val-Ile-
Leu-Gly-Lys-His-Pro-Asn-Ser-Phe-Ile-Cys-Leu-Lys-Arg-Leu-
Pro-Ile-Gly-Ser-Tyr-Ile-Asp-(COOH),
and substantially pure polypeptides that are substantially
equivalent and substantially homologous thereto is disclosed.
Pharmaceutical compositions containing these polypeptides
and methods to use them are also disclosed.

EP 0 336 760 A2

Bundesdruckerei Berlin

**Description**

## BONE-INDUCING PROTEIN

Technical Field

The present invention relates to protein chemistry and osteoplasty. More particularly, it relates to a protein that induces bone growth, implant and pharmaceutical compositions containing that protein, methods for promoting bone growth using such compositions, methods for stimulating bone marrow progenitor cells to divide and differentiate into bone marrow cells, and methods for treating diseases associated with dysfunction/malfunction of bone generation and/or bone resorption, such as osteoporosis.

Background Art

It has been established that bone contains materials which can stimulate the formation of new bone when placed in contact with living systems. (Urist, M. R., Clin Orthop (1968) 56:37; Science (1965) 150:893; Reddi, A. H., et al., Proc Natl Acad Sci (USA) (1972) 69:1601.) Attempts have been made to purify whatever factors are responsible for this activity. A "bone morphogenic protein" (BMP) was extracted from demineralized bone using urea or guanidine hydrochloride and reprecipitated according to the disclosures in U.S. Patents Nos. 4,294,753 and 4,455,256 to Urist. Urist subsequently reported (Urist, M. R., Clin Orthop Rel Res (1982) 162:219) that ion exchange purification of this crude protein mixture yielded an activity which was unadsorbed to carboxymethyl cellulose resin (CMC) at pH 4.8. Urist's reports in Science (1983) 220:680-685 and Proc Natl Acad Science (USA) (1984) 81:371-375 describe BMPs having molecular weights of 17,500 and 18,500 daltons. Urist's patent publication, EPA Publication No. 0212474, describes BMP fragments of 4,000 to 7,000 daltons obtained by limited proteolysis of BMP.

U.S. Patent No. 4,608,199 describes a bone-derived protein of 30,000-32,000 daltons. The protein is described as being water soluble and having no affinity for concanavalin A (ConA).

WO 88/00205 reports four proteins, designated BMP-1, BMP-2 Class I, BMP-2 Class II and BMP-3, that are alleged to have osteogenic activity by themselves or in combination with other factors. Sequences are provided for each of these proteins which show no homology to the sequence (see below) of the osteogenic protein of the present invention.

U.S. 4,434,094 to Seyedin and Thomas reported the partial purification of a bone generation-stimulating, bone-derived protein by extraction with chaotropic agents, fractionation on anion and cation exchange columns, and recovery of the activity from a fraction adsorbed to CMC at pH 4.8. This new protein fraction was termed "osteogenic factor" (OF) and was characterized as having a molecular weight below about 30,000 daltons.

Commonly owned U.S. Patent No. 4,774,332 describes two proteins that were purified to homogeneity using a purification procedure that is similar in part to that disclosed in U.S. 4,434,094. Those two proteins eluted from CMC at about a 150-200 mM NaCl gradient. These two proteins were originally called cartilage-inducing factor (CIF) A and CIF B. CIF A was subsequently found to be identical to a previously identified protein called transforming growth factor beta (TGF-beta). CIF B has been found to be a novel form of TGF-beta and is now known as TGF-beta2.

Commonly owned U.S. Patent No. 4,627,982 concerns a partially purified bone-inducing factor present in the CMC-bound fraction of U.S. 4,434,094 that elutes in the portion of the NaCl gradient below that in which the major portions of CIF A and CIF B elute (i.e., below about 150 mM NaCl). The present invention relates to the identification of the active ingredient of that fraction. In this regard, at the time that patent was filed it was not known whether the bone-inducing activity was attributable to a single protein or a plurality of proteins acting in concert. Identification of the protein(s) responsible for bone-inducing activity was complicated by the large number of proteins in the fraction (estimated to be several hundred), the lack of a conclusive in vitro assay for bone-inducing activity, and extensive difficulty in isolating the active protein from other proteins in the fraction. Indeed, it has taken applicants approximately three years of effort--in which a variety of protein fractionation procedures were attempted--to obtain the bone-inducing protein from the CMC-bound fraction at a level of purity at which it could be sequenced and identified as being responsible for the activity observed in the crude fraction.

As discussed in detail below, the bone-inducing activity of the fraction has been found to be attributable to a glycoprotein component having a variable (apparently due to variation in glycosylation) molecular weight (in the glycosylated state) of approximately 20,000-28,000 daltons as determined by sodium dodecyl sulfate polyacrylamide gel electrophoresis (SDS-PAGE) analysis. Amino acid sequencing of the active component indicates it is composed of a protein that has a sequence different from any previously reported sequence.

Disclosure of the Invention

The invention relates to a substantially pure polypeptide that induces bone formation in vivo in mammals.

Accordingly, one aspect of the invention is a substantially pure osteogenically active polypeptide having an internal sequence selected from the group consisting of

　　a) -Lys-Tyr-Asn-Lys-Ile-Lys-Ser-Arg-Glu-Ile-Lys-Ala-Asn-Thr-Phe-Lys-Lys-Leu-His-Asn-Leu-Ser-Phe-Leu-Tyr-Leu-Asp-His-Asn-Ala-Leu-Glu-;

　　b) -Leu-His-Asn-Leu-Ser-Phe-Leu-Tyr-Leu-Asp-His-Asn-Ala-Leu-Glu-Ser-Val-Pro-Leu-Asn-Leu-Pro-Glu-;

c) -Ser-Leu-Arg-Val-Ile-His-Leu-Gln-Phe-Asn-Asn-Ile-Thr-Ser-Ile-Thr-Asp-Asp-Thr-Phe-Cys-Lys-Ala-;

d) -Ala-Asn-Asp-Thr-Ser-Tyr-Ile-Arg-Asp-Arg-Ile-Glu-Glu-Ile-Arg-Leu-Glu-Gly-Asn-Pro-Val-Ile-;

e) -Gly-Asn-Pro-Val-Ile-Leu-Gly-Lys-His-Pro-Asn-Ser-Phe-Ile-Cys-Leu-Lys-Arg-Leu-Pro-Ile-Gly-Ser-Tyr-; and/or a carboxy terminal sequence as follows

-Arg-Leu-Pro-Ile-Gly-Ser-Tyr-Ile-Asp-(COOH),

and substantially pure polypeptides that are substantially equivalent and substantially homologous thereto.

Another aspect of the invention is a substantially pure osteogenically active polypeptide having the following amino acid sequence:
(H$_2$N)-Ala-Lys-Tyr-Asn-Lys-Ile-Lys-Ser-Arg-Gly-Ile-Lys-Ala-Asn-Thr-Phe-Lys-Lys-Leu-His-Asn-Leu-Ser-Phe-Leu-Tyr-Leu-Asp-His-Asn-Ala-Leu-Glu-Ser-Val-Pro-Leu-Asn-Leu-Pro-Glu-Ser-Leu-Arg-Val-Ile-His-Leu-Gln-Phe-Asn-Asn-Ile-Thr-Ser-Ile-Thr-As p-Asp-Thr-Phe-Cys-Lys-Ala-Asn-Asp-Thr-Ser-Tyr-Ile-Arg-Asp-Arg-Ile-Glu-Glu-Ile-Arg-Leu-Glu-Gly-Asn-Pro-Val-Ile-Leu-Gly-Lys-His-Pro-Asn-Ser-Phe-Ile-Cys-Leu-Lys-Arg-Leu-Pro-Ile-Gly-Ser-Tyr-Ile-Asp-(COOH),

and substantially pure polypeptides that are substantially equivalent and substantially homologous thereto.

Another aspect of the invention is a composition for inducing bone formation or bone marrow cell production in vivo comprising (a) an effective amount of one or more of the above-described osteogenically active polypeptides and (b) an effective amount of TGF-beta combined with (c) a pharmaceutically acceptable carrier.

Another aspect of the invention is a method of inducing bone formation in vivo at a desired site comprising implanting the above described composition in a mammal at said site.

Yet another aspect of the invention is a method of inducing bone marrow cell production in a living mammal comprising administering systemically to the mammal an effective amount of one or more of the above described polypeptides.

Another aspect of the invention is a method of treating an individual for a condition characterized by insufficient bone formation and/or undesired bone resorption comprising administering systemically to the individual an effective amount of one or more of the above-described polypeptides.

Still another aspect of the invention is an improvement in a method for treating a living mammal for cancer of the hematopoietic system wherein the mammal is subjected to irradiation to kill neoplastic hematopoietic cells the improvement comprising administering systemically to the mammal prior to irradiation a sufficient amount of TGF-beta to suppress hematopoietic stem cell division and administering systemically to the mammal after irradiation a sufficient amount of one or more of the above described polypeptides to stimulate hematopoietic stem cell division.

Another aspect is a method for isolating a substantially pure osteogenically active protein composition from demineralized bone comprising:

(a) extracting demineralized bone with a chaotropic (dissociative) extractant that solubilizes nonfibrous proteins;

(b) subjecting the extract from step (a) to gel filtration to recover a fraction containing proteins of molecular weight of approximately 20,000-36,000 daltons;

(c) adsorbing the fraction from step (b) onto a carboxymethyl cellulose cation exchanger at approximately pH 4.5-5.5 under denaturing conditions;

(d) eluting a fraction from the cation exchanger with a sodium chloride gradient of about 10 mM to about 150 mM;

(e) adsorbing the eluate of step (d) onto a cross-linked ConA column;

(f) eluting bound protein from the column of step (e);

(g) adsorbing the eluate of step (f) onto a heparin-sepharose column;

(h) eluting bound protein from the column of step (g); and

(i) chromatographing the eluate of step (h) on an RP-HPLC column using a trifluoroacetic acid- acetonitrile system and recovering the substantially pure osteogenically active protein composition as the fraction eluting from the column at approximately 42-45% acetonitrile.

Further aspects of the invention are recombinant materials (i.e., recombinant DNA, recombinant vectors, and recombinant cells or microorganisms) and processes for producing the osteogenic proteins of the invention.

Brief Description of the Drawings
In the drawings:

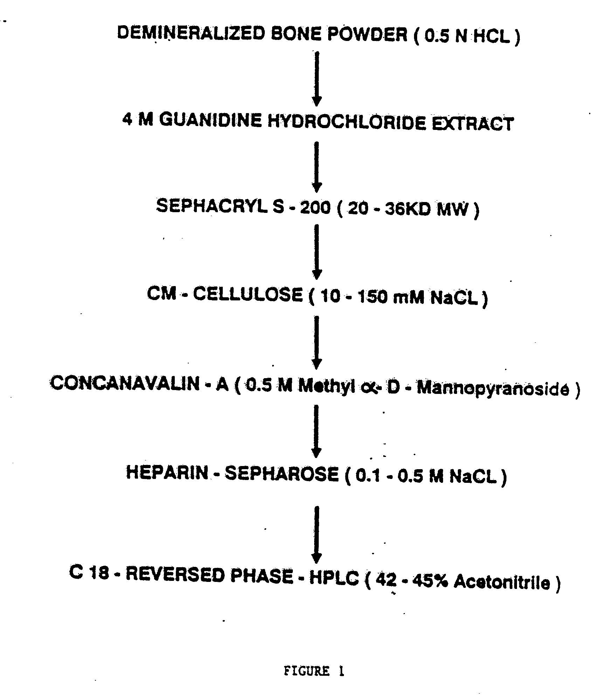

Figure 1 is a flow chart of the process that was used to isolate osteogenic protein from demineralized bovine bone.

Figure 2 is a graph of the optical densities (absorbances at 280 nm) of the gel filtration fractions of the gel filtration fractions of the example (©C).

Figure 3 is a graph of the optical densities (absorbances at 280 nm) of eluate fractions from the preparative ion exchange chromatography of the example (©D).

Figure 4 is a graph of the optical densities (absorbances at 280 nm) of eluate fractions from the cross-linked ConA chromatography step of the example (©E);

Figure 5 is a graph of the optical densities (absorbances at 280 nm) of eluate fractions from the heparin-sepharose chromatography step of the example (©F); (absorbances at 230 nm) of the gradient fractions from the

Figure 6 is a graph of the optical densities (absorbances at 230 nm) of the gradient fractions from the C18-RP-HPLC chromatography step of the example (©G);

Figure 7 is a table showing results of amino acid sequencing of the osteogenically active isolate of the invention and locations of the sequenced fragments in the overall sequence.

Figure 8 is a photograph of an autoradiograph of SDS-PAGE analyses of the purified osteogenic protein that are described in the example (©H) (lanes A and C show glycosylated protein; lanes B and D show enzymatically deglycosylated protein).

Figure 9 is a bar graph showing the results of the assays described in the example (©I.3 and I.4).

Figure 10 is a bar graph of the results of the assays described in ©J.1. of the examples.

Figure 11 is a graph of the results of the tests described in ©J.3. of the examples.

## Modes of Carrying Out the Invention

### Isolation of Bone-Inducing Protein from Bone

In view of the showing that bone inductive proteins from human, monkey, bovine and rat are nonspecies specific in their ability to produce endochondral bone in xenogeneic implants (Sampath, T.K., et al, Proc Natl Acad Sci (USA) (1983) 80:6591) it is believed that the bovine protein described herein has been highly conserved among mammalian species--i.e., corresponding bone-inducing proteins from different mammalian species (herein called "species analogs") will have substantially homologous amino acid sequences that vary from the bovine protein, if at all, in one or more amino acid residue additions, deletions or substitutions and/or substantially similar glycosylation patterns that do not affect the nonspecies-specific ability of the molecule to induce bone formation. In this regard, the terms "substantially equivalent" and "substantially homologous" are intended to mean proteins, regardless of species or method of preparation, that have the same amino acid sequence as the bovine osteogenic protein described in the examples and proteins of similar but different amino acid sequence, which difference(s) does not affect nonspecies-specific endochondral bone- inducing activity adversely. The amino acid sequences of such "substantially homologous" proteins will usually be at least 50% homologous, more usually at least 80% homologous, and preferably at least 90% homologous to the bovine sequence described herein. Accordingly, such proteins may be derived from bone of diverse mammalian origin or synthesized using recombinant DNA procedures. The term is intended to include muteins or analogs of the native protein that are altered in manners known in the art, such as by substitution of cysteines that are not essential for activity with neutral (uncharged) amino acids to avoid improper disulfide bonding, by substitution or elimination of residues in the asparagine-linked glycosylation sites of the proteins to alter glycosylation patterns, by substitution of methionines that are not necessary for activity to make the molecules less susceptible to oxidation, by chemical modification of one or more residues, or by elimination or alteration of side-chain sugars. The source of protein prepared by purification from native sources is advantageously porcine or bovine long bone because of its ready availability.

The process for isolating the osteogenic protein from bone is as follows. The bone is first cleaned using mechanical or abrasive techniques, fragmented, and further washed with, for example, dilute aqueous acid preferably at low temperature. The bone is then demineralized by removal of the calcium phosphates in their various forms, usually by extraction with stronger acid. These techniques are understood in the art, and are disclosed, for example, in U.S. 4,434,094. The resulting preparation, a demineralized bone, is the starting material for the preparation of the claimed osteogenic protein from native sources.

The initial extraction is designed to remove the nonfibrous (e.g., noncollagenous) proteins from the demineralized bone. This can be done with the use of chaotropic agents such as guanidine hydrochloride (at least about 4 molar), urea (8 molar) plus salt, or sodium dodecylsulfate (at least about 1% by volume) or such other chaotropic agents as are known in the art (Termine et al., J Biol Chem (1980) 255:9760-0772; and Sajera and Hascall, J Biol Chem (1969) 244:77-87 and 2384-2396). The extraction is preferably carried out at reduced temperatures to reduce the likelihood of digestion or denaturation of the extracted protein. A protease inhibitor may be added to the extractant, if desired. The pH of the medium depends upon the extractant selected. The process of extraction generally takes on the order of about 4 hr to 1 day.

After extraction, the extractant may be removed by suitable means such as dialysis against water, preceded by concentration by ultrafiltration if desired. Salts can also be removed by controlled electrophoresis, or by molecular sieving, or by any other means known in the art. It is also preferred to maintain a low temperature during this process so as to minimize denaturation of the proteins. Alternatively, the extractant chaotropic agent need not be removed, but rather the solution need only be concentrated, for example, by ultrafiltration.

The extract, dissolved or redissolved in chaotropic agent, is subjected to gel filtration to obtain fractions of molecular weight in the range of about 20,000 to 36,000 daltons. Gel sizing is done using standard techniques, preferably on a Sephacryl S-200 column at room (10°C-25°C) temperature.

The sized fraction is then subjected to ion exchange chromatography using CMC at approximately pH 4.5-5.2 preferably about 4.8, in the presence of a nonionic chaotropic agent such as 6 M urea. Other cation exchangers may be used, including those derived from polyacrylamide and cross-linked dextran; however cellulosic cation exchangers are preferred. Of course, as in any ion exchange procedure, the solution must be freed of competing ions before application to the column. The factor is adsorbed on the column and is eluted in an increasing salt concentration gradient in the range of about 10 mM to about 150 mM. This fraction is designated "CMB-1" for convenience.

CMB-1 is lyophilized and the dry CMB-1 is dissolved in aqueous sodium deoxycholate (DOC), pH 8.0. This solution is affinity chromatographed on an equilibrated column of ConA cross-linked to resin. The ConA-bound material is eluted from the resin with aqueous DOC containing a displacement carbohydrate. This fraction is designated "CAB-1" for convenience.

CAB-1 is reequilibrated for heparin-sepharose chromatography by desalting on a GH-25 column equilibrated on heparin-sepharose buffer, 6 M urea, 0.1 M NaCl, 50 mM Tris-HCl pH 7.2. The desalted fraction is loaded into a heparin-sepharose column. After washing, bound material is eluted from the column using the same buffer at a 0.5 M NaCl salt concentration. The resulting eluate is designated "HSB-1" for convenience.

HSB-1 is diluted and adjusted to pH 2 and loaded onto a C18-RP-HPLC column. Bound proteins were gradient eluted from the column using a solvent consisting of 90% acetonitrile in 0.1% aqueous TFA (Solvent B). The osteogenic protein of the invention elutes at approximately 47-50% of solvent B (42-45% acetonitrile) by volume.

Proteins eluted by the C18 chromatography were iodinated by the chloramine-T method. Analysis of the fraction by SDS-PAGE and autoradiography shows a major broad band at 20,000 to 28,000 daltons comprising the osteogenic protein. The "smearing" of the protein is believed to mainly be the result of heterogeneity in the glycosylation of the molecule or perhaps variable post-translational modification or proteolytic degradation. After enzymatic or chemical deglycosylation, SDS-PAGE analysis of the protein gives a single band of approximately 9600 daltons. Reduction of the deglycosylated protein with dithiothreitol does not affect its migration.

Initial amino acid sequence analysis of the glycosylated protein yielded the following internal sequence in the N-terminal portion of the protein:
-Lys-Tyr-Asn-Lys-Ile-Lys-Ser-Arg-Gly-Ile-Lys-Ala-Asn-Thr-Phe-Lys-Lys-Leu-His-Asn-Leu-Ser-Phe-X-Tyr-Thr-Asp-His-Asn-Ala-Leu-Glu-

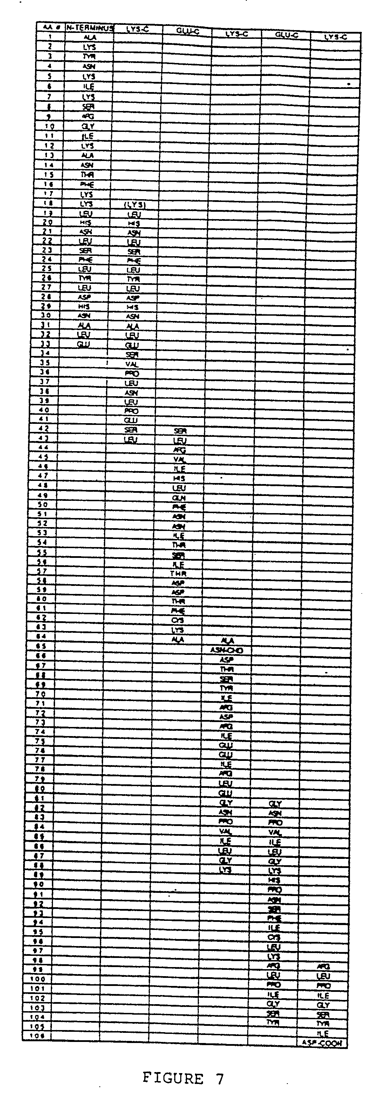

The initial amino acid (Lys) in the above sequence is nearest to the N-terminal. Initially, the nature of the signal obtained for the residue designated X did not permit this residue to be identified. Repeated sequencing and sequencing of endoproteinase Lys-C (an enzyme that cleaves proteins at Lys residues) and endoproteinase Glu-C (an enzyme that cleaves proteins at Glu residues) digests have revealed that the above sequence is preceded by an Ala residue which is the N-terminus, that the residue designated X is Leu, that the second Thr residue (the 26th residue in the above sequence) was incorrect and that this residue is actually a Leu residue, and that the isolate consists of a protein of approximately 106 amino acids. Figure 7 provides a summary of these sequence analyses. The symbol "CHO" designates a carbohydrate substituent. The symbol "COOH" represents a carboxyl group and designates the carboxy terminus. The first column (on the left) provides the sequence analysis of the N-terminal fragment described above. The second, fourth, and sixth columns give the sequences of three major Lys-C fragments of the isolate. The third and fifth columns give the sequences of two Glu-C fragments.

The positioning of the fragments shown in Fig. 7 are based on the apparent overlap in the various fragments. Based on this positioning, the most likely sequence for the protein is:
(H_2N)-Ala-Lys-Tyr-Asn-Lys-Ile-Lys-Ser-Arg-Gly-Ile-Lys-Ala-Asn-Thr-Phe-Lys-Lys-Leu-His-Asn-Leu-Ser-Phe-Leu-Tyr-Leu-Asp-His-Asn-Ala-Leu-Glu-Ser-Val-Pro-Leu-Asn-Leu-Pro-Glu-Ser-Leu-Arg-Val-Ile-His-Leu-Gln-Phe-Asn-Asn-Ile-Thr-Ser-Ile-Thr-Asp-Asp-Thr-Phe-Cys-Lys-Ala-Asn-Asp-Thr-Ser-Tyr-Ile-Arg-Asp-Arg-Ile-Glu-Glu-Ile-Arg-Leu-Glu-Gly-Asn-Pro-Val-Ile-Leu-Gly-Lys-His-Pro-Asn-Ser-Phe-Ile-Cys-Leu-Lys-Arg-Leu-Pro-Ile-Gly-Ser-Tyr-Ile-Asp-(COOH),

It will be appreciated that this sequence is not conclusive due to the nature of the analysis technique. Accordingly, the sequences given above may not be entirely accurate. The sequence may be confirmed by identifying the gene for the protein, sequencing the gene and deducing the amino acid sequence therefrom.

Amino acid composition analyses of the isolated deglycosylated protein were carried out with and without performic acid oxidation (performic acid oxidation permits detection of cysteic acid residues). The results of these analyses are indicated below.

| Amino Acid | Without Performic Oxidation Integer Res/Mol | With Performic Oxidation Integer Res/Mol |
|---|---|---|
| Asx[1] | 11 | 12 |
| Thr | 4 | 4 |
| Ser | 6 | 7 |
| Glx[2] | 8 | 7 |
| Gly | 5 | 8 |
| Ala | 4 | 4 |
| Val | 4 | 3 |
| Ile | 6 | 6 |
| Leu | 9 | 9 |
| Tyr | 3 | ND |
| Phe | 3 | 3 |
| Lys | 7 | 6 |
| His | 3 | 3 |
| Arg | 5 | 5 |
| Pro | 5 | 4 |
| Met | 0 | 0 |
| Cys | ND | 2 |
| TOTAL | *83 | 83(86[3]) |

[1]Includes both Asp and Asn
[2]Includes both Glu and Gln

ND = not determined

[3]Includes three Tyr residues
*based on SDS-PAGE determined molecular weight of approximately 9600

To determine the amino acid composition of the protein, the protein was hydrolyzed for 24 hr at 110°C in 6 N HCl containing 0.1% phenol. To determine the presence of Cys residues, performic acid oxidation was done prior to hydrolysis. Amino acids were detected on a Beckman 6300 analyzer using ninhydrin detection. It should be understood that these compositions are only an approximation due to the limitations of the analytical technique. Further it is clear that the molecular weight determined by SDS-PAGE does not conform with the molecular weight projected from the amino acid sequence analyses and is substantially lower than that projection. The invention provides the osteogenic protein in substantially pure form in which it is essentially free of other molecules with which it is associated in nature. In this regard, the term "substantially pure" intends a composition containing less than about 30% by weight contaminating protein, preferably less than about 10% contaminating protein, and most preferably less than about 5% by weight contaminating protein. The term "substantially pure" is used relative to proteins with which the osteogenic protein is associated in nature and is not intended to exclude compositions in which the osteogenic protein is admixed with nonproteinaceous pharmaceutical carriers or vehicles or proteinaceous pharmaceutical carriers or vehicles. The invention also provides the osteogenic protein in novel partially glycosylated or totally deglycosylated form (both of which are referred to herein as "deglycosylated"). The term "osteogenic" intends the ability to induce new bone formation either alone or in combination with a co-factor. Assays for osteogenic activity are described in the examples, infra.

Further characterization of the osteogenic protein of this invention may be carried out using procedures known in the art. Its isoelectric focusing pattern, isoelectric point, susceptibility to degradation by proteases or other chemicals such as acids or bases, and affinity to other materials such as other lectins, and the like may be so determined.

Based on the above amino acid sequence, oligonucleotide probes which contain the codons for a portion or all of the determined amino acid sequence are prepared and used to screen DNA libraries for genes encoding the osteogenic protein and substantially homologous genes that encode related proteins having osteogenic activity. The homologous genes may be from other species of mammals or animals (e.g., avians) or may represent other members of a family of related genes. The basic strategies for preparing oligonucleotide probes and DNA libraries, as well as their screening by nucleic acid hybridization, are well known to those of ordinary skill in the art. See, e.g., DNA CLONING: VOLUME I (D.M. Glover ed. 1985); NUCLEIC ACID HYBRIDIZATION (B.D. Hames and S.J. Higgins eds. 1985); OLIGONUCLEOTIDE SYNTHESIS (M.J. Gate ed. 1984); T. Maniatis, E.F. Frisch & J. Sambrook, MOLECULAR CLONING: A LABORATORY MANUAL (1982).

First, a DNA library is prepared. Since the identified protein is bovine, it is logical to probe a bovine library first, find full length clones and use the full length bovine clones to probe libraries of other mammalian species to identify the osteogenic protein gene (and thus the amino acid sequences) of other species. The library can consist of a genomic DNA library. Bovine and human genomic libraries are known in the art. See, e.g., Lawn et al., Cell (1978) 15:1157-1174. DNA libraries can also be constructed of cDNA prepared from a poly-A RNA (mRNA) fraction by reverse transcription. See, e.g., U.S. Patent Nos. 4,446,235; 4,440,859; 4,433,140; 4,431,740; 4,370,417; 4,363,877. The mRNA is isolated from an appropriate cell line or tissue that expresses the factor. Libraries from cells involved in bone formation (e.g., osteoblasts) or from osteotumors (e.g., osteosarcoma lines) are likely sources to probe for the osteogenic protein nucleic acids. Representative examples of such cell lines are the human amniotic line WISH (ATCC CCL25), the human osteosarcoma lines TE-85 (ATCC CRL1547) and MG63 (ATCC CRL1427), the human prostate carcinoma line PC-3 (ATCC CRL1435), the rat osteosarcoma line UMR-106 (ATCC CRL1661) and the mouse osteosarcoma line DUNN (available from Dr. Vital Ghanta, University of Alabama Medical School). cDNA (or genomic DNA) is cloned into a vector suitable for construction of a library. A preferred vector is a bacteriophage vector, such as phage lambda. The construction of an appropriate library is within the skill of the art.

Once the library is constructed, oligonucleotides to probe the library are prepared and used to isolate the desired osteogenic protein genes. The oligonucleotides are synthesized by any appropriate method. The particular nucleotide sequences selected are chosen so as to correspond to the codons encoding the known amino acid sequences of the osteogenic protein. Since the genetic code is redundant, it will often be necessary to synthesize several oligonucleotides to cover all, or a reasonable number, of the possible nucleotide sequences which encode a particular region of a protein. Thus, it is generally preferred in selecting a region upon which to base the probes, that the region not contain amino acids whose codons are highly degenerate. It may not be necessary, however, to prepare probes containing codons that are rare in the mammal from which the library was prepared. In certain circumstances, one of skill in the art may find it desirable to prepare probes that are fairly long, and/or encompass regions of the amino acid sequence which would have a high degree of redundancy in corresponding nucleic acid sequences, particularly if this lengthy and/or redundant region is highly characteristic of the protein. Probes covering the complete gene, or a substantial part of the genome, may also be appropriate, depending upon the expected degree of homology. Such would be the case, for example, if a cDNA of a bovine osteogenic protein was used to screen a human gene library for the corresponding human osteogenic protein. It may also be desirable to use two probes (or sets of probes), each to different regions of the gene, in a single hybridization experiment. Automated oligonucleotide synthesis has made the preparation of large families of probes relatively straightforward. While the exact length of the probe employed is not critical, generally it is recognized in the art that probes from about 14 to about 20 base pairs are usually effective. Longer probes of about 25 to about 60 base pairs are also used.

The selected oligonucleotide probes are labeled with a marker, such as a radionucleotide or biotin using standard procedures. The labeled set of probes is then used in the screening step, which consists of allowing the single-stranded probe to hybridize to isolated ssDNA from the library, according to standard techniques. Either stringent or permissive hybridization conditions could be appropriate, depending upon several factors, such as the length of the probe and whether the probe is derived from the same species as the library, or an evolutionarily close or distant species. The selection of the appropriate conditions is within the skill of the art. See generally, NUCLEIC ACID HYBRIDIZATION, supra. The basic requirement is that hybridization conditions be of sufficient stringency so that selective hybridization occurs; i.e., hybridization is due to a sufficient degree of nucleic acid homology (e.g., at least about 75%), as opposed to nonspecific binding. Once a clone from the screened library has been identified by positive hybridization, it can be confirmed by restriction enzyme analysis and DNA sequencing that the particular library insert contains a gene for the osteogenic protein.

Alternatively, a DNA coding sequence for an osteogenic protein can be prepared synthetically from overlapping oligonucleotides whose sequence contains codons for the amino acid sequence of the protein. Such oligonucleotides are prepared by standard methods and assembled into a complete coding sequence. See, e.g., Edge, Nature (1981) 292:756; Nambair et al., Science (1984) 223:1299; Jay et al., J Biol Chem (1984) 259:6311.

Accordingly recombinant polynucleotides that encode the osteogenic polypeptides may be prepared and isolated by one or more of the above described techniques. The term "recombinant polynucleotide" as used herein denotes a polynucleotide of genomic, cDNA, semisynthetic or synthetic origin which, by virtue of its origin or manipulation (1) is not associated with all or a portion of the nucleic acid with which it is associated in nature or in the form of a library (2) is linked to a polynucleotide to which it is not linked in nature or (3) is not found in nature.

Recombinant DNA molecules containing the coding sequence for the osteogenic protein can be cloned in any suitable vector and thereby maintained in a composition substantially free of vectors that do not contain the coding sequence of the osteogenic protein (e.g., other library clones). Numerous cloning vectors are known to those of skill in the art, and the selection of an appropriate cloning vector is a matter of choice. Examples of recombinant DNA vectors for cloning and the host cells which they transform include bacteriophage lambda (E. coli), pBR322 (E. coli), pACYC177 (E. coli), pKT230 (gram-negative bacteria), pGV1106 (gram-negative bacteria), pLAFR1 (gram-negative bacteria), pME290 (non-E. coli gram-negative bacteria), pHV14 (E. coli and Bacillus subtilis), pBD9 (Bacillus), pIJ61 (Streptomyces), pUC6 (Streptomyces), actinophage C31 (Streptomyces), Ylp5 (yeast), YCp19 (yeast), and bovine papilloma virus (mammalian cells). See generally, DNA CLONING: VOLUMES I & II, supra; MOLECULAR CLONING: A LABORATORY MANUAL, supra.

In one embodiment of the present invention, the coding sequence for an osteogenic protein gene is placed under the control of a promoter, ribosome binding site (for bacterial expression) and, optionally, an operator (collectively referred to herein as "control" sequences), so that the DNA sequence encoding the osteogenic protein (referred to herein as the "coding" sequence) is transcribed into RNA in the host cell transformed by the vector. The coding sequence may or may not contain a signal peptide or leader sequence. The determination of the point at which the mature protein begins and the signal peptide ends is easily determined from the N-terminal amino acid sequence of the mature protein. The osteogenic protein can also be expressed in the form of a fusion protein, wherein a heterologous amino acid sequence is expressed at the N-terminal. See e.g., U.S. Patents Nos. 4,431,739; 4,425,437.

The recombinant vector is constructed so that the osteogenic protein coding sequence is located in the vector with the appropriate control sequences, the positioning and orientation of the osteogenic protein coding sequence with respect to the control sequences being such that the coding sequence is transcribed under the control of the control sequences (i.e., by RNA polymerase which attaches to the DNA molecule at the control sequences). The control sequences may be ligated to the coding sequence prior to insertion into a vector, such as the cloning vectors described above. Alternatively, the coding sequence can be cloned directly into an expression vector which already contains the control sequence and an appropriate restriction site downstream from control sequences. For expression of the osteogenic protein coding sequence in procaryotes and yeast, the control sequences will be heterologous to the coding sequence. If the selected host cell is a mammalian cell, the control sequences can be heterologous or homologous to the osteogenic protein coding sequence, and the coding sequence can be genomic DNA, cDNA or synthetic DNA. Either genomic or cDNA coding sequence may be expressed in yeast. If glycosylation similar to the native molecule is desired, the gene may be expressed in yeast or mammalian cells (COS, CHO, or CV-1 cells) using vectors and procedures known in the art. In this regard, initial tests at selected concentrations indicate tentatively that the totally deglycosylated protein is not active. For this reason, expression in eukaryotes that are capable of effecting glycosylation may be essential to make an active protein by recombinant procedures.

A number of procaryotic expression vectors are known in the art. See, e.g., U.S. Patent Nos. 4,440,859; 4,436,815; 4,431,740; 4,431,739; 4,428,941; 4,425,437; 4,418,149; 4,411,994; 4,366,246; 4,342,832. See also British Patent Specifications GB 2,121,054; GB 2,008,123; GB 2,007,675; and European Patent Specification 103,395. Yeast expression vectors are known in the art. See, e.g., U.S. Patent Nos. 4,446,235; 4,443,539; 4,430,428. See also European Patent Specifications 103,409; 100,561; and 96,491.

Recombinant osteogenic protein can be produced by growing host cells transformed by the expression vector described above under conditions whereby the osteogenic protein is produced. The osteogenic protein is then isolated from the host cells and purified. If the expression system secretes osteogenic protein into growth media, the protein can be purified directly from cell-free media. If the recombinant protein is not secreted, it is isolated from cell lysates. The selection of the appropriate growth conditions and recovery methods are within the skill of the art or are apparent from the recovery methods used to isolate the native osteogenic proteins. The recombinant protein may be recovered by affinity chromatography using the antibodies produced in accordance with the invention. Recombinant osteogenic protein may be unglycosylated or have a different glycosylation pattern than the native molecule depending upon the host that is used to produce it. As indicated above, the deglycosylated protein may not be active. It would be useful, however, for making antibodies that recognize sequential epitopes of the protein.

Either native, deglycosylated, or synthetic (recombinant) osteogenic protein can be used to produce antibodies, both polyclonal and monoclonal. The term "antibody" is intended to include whole Ig of any isotype or species as well as antigen binding fragments and chimeric constructs. If polyclonal antibodies are desired, purified osteogenic protein is used to immunize a selected mammal (e.g., mouse, rabbit, goat, horse, etc.) and serum from the immunized animal later collected and treated according to known procedures. Compositions containing polyclonal antibodies to a variety of antigens in addition to the osteogenic protein can be made substantially free of antibodies which are not anti-osteogenic protein antibodies by passing the composition through a column to which osteogenic protein has been bound. After washing, polyclonal antibodies to the osteogenic protein are eluted from the column. Monoclonal anti-osteogenic protein antibodies can also be readily produced by one skilled in the art. The general methodology for making monoclonal antibodies by hybridomas is well known. Immortal, antibody-producing cell lines can also be created by techniques other than fusion, such as direct transformation of B lymphocytes with oncogenic DNA, or transfection with Epstein-Barr virus. See, e.g., M. Schreier et al., HYBRIDOMA TECHNIQUES (1980); Hammerling et al., MONOCLONAL ANTIBODIES AND T-CELL HYBRIDOMAS (1981); Kennett et al., MONOCLONAL ANTIBODIES (1980).

By employing osteogenic protein (native, deglycosylated or synthetic) as an antigen in the immunization of the source of the B-cells immortalized for the production of monoclonal antibodies, a panel of monoclonal antibodies recognizing epitopes at different sites on the osteogenic protein molecule can be obtained. Antibodies which recognize an epitope in the binding region of the protein can be readily identified in competition assays between antibodies and protein. Antibodies which recognize a site on the osteogenic protein are useful, for example, in the purification of the protein from cell lysates or fermentation media, in characterization of the protein and in identifying immunologically related proteins. Such immunologically related proteins (i.e., that exhibit common epitopes with the osteogenic protein) are another aspect of the invention. In general, as is known in the art, the antiosteogenic protein antibody is fixed (immobilized) to a solid support, such as a column or latex beads, contacted with a solution containing the osteogenic protein, and separated from the solution. The osteogenic protein, bound to the immobilized antibodies, is then eluted.

Osteogenic Compositions

The osteogenic protein of the invention may be used to induce de novo bone formation in circumstances where bone is not normally formed. The protein may thus be used prophylactically to reduce the likelihood of fracture, improve fixation of artificial joints, repair congenital or trauma-induced bone defects, or in cosmetic plastic surgery. The protein may also be used to enhance bone formation in instances where bone is normally formed, such as in fracture repair, replacement of surgically removed bone, or repair of bone damaged by periodontal disease or in other tooth or alveolar ridge repair processes. In such uses, the protein will be administered locally, such as by implantation, at the desired site of bone formation.

The protein may also be administered systemically, such as intravenously, to treat indications associated with insufficient bone formation and/or undesirable levels of bone resorption such as localized, regionalized or generalized osteoporosis or to stimulate bone marrow progenitor cells in the treatment of malfunctions or dysfunctions of the hematopoietic system such as chronic and acute mylocytic leukemia and other cancers of the hematopoietic system or in post-irradiation treatment to stimulate bone marrow stem cells to divide and differentiate. In this regard TGF-beta may be used pre-irradiation to suppress marrow stem cell reproduction and differentiation and the invention protein may be used post-irradiation to stimulate such cells.

Initial tests of the osteogenic protein composition indicate that it is necessary, in the concentrations and formulations tested, to coadminister a protein having TGF-beta activity to achieve bone induction at nonbony sites. It may be that TGF-beta induces proliferation of bone forming cells and the osteogenic protein of the invention induces differentiation of such cells. In this regard, TGF-beta (TGF-beta1, TGF-beta2, other members of the TGF-beta family, or mixtures thereof) may enhance the process of bone induction through ancillary activities such as antiinflammatory activity, chemotactic activity, and the like. Other molecules that exhibit such activities may also be useful as co-factors for bone-induction. The protein may, of course, be active at other concentrations or in other formulations. Further it may not be necessary to coadminister TGF-beta at bony sites since the amount of endogenous TGF-beta present at the site of action or in systemic applications may be sufficient.

The osteogenic protein of the invention will normally be formulated in osteogenically effective amounts with pharmaceutically acceptable solid or fluid carriers, for local injection or implantation at the desired site of activity or systemic administration by conventional parenteral routes. Preferably the formulations for local administration include a matrix material that is capable of presenting the protein at the desired site of activity as well as providing a structure for developing bone and cartilage. Potential matrices may be biodegradable or nonbiodegradable and be chemically or biologically defined. Examples of such materials are calcium sulfate, hydroxyapatite, tricalciumphosphate, polyorthoesters, polylactic-polyglycolic acid copolymers, collagen, bioglass, and the like. Formulations for systemic administration will typically involve liquid vehicles that are commonly used for parenteral administration of proteinaceous therapeutics.

The osteogenic protein of the invention may be conjugated with other molecules to increase its water-solubility, increase its half-live, or enhance its ability to bind to bone. For instance, it may be conjugated to polyethylene glycol to increase its water solubility or to bone-binding molecules such as bisphosphonates (e.g. 1-hydroxyethylidene-1,1-bisphosphonic acid, dichloromethylene bisphosphonic acid, and 3-amino-1-hydroxypropylidene-1,1-bisphosphonic acid) and fluorochromes (e.g. tetracyclines, calcein blue, xylenol orange, calcein green, and alizarin complexone red) to target the protein to bony sites. Various agents for conjugating molecules to proteins are well known in the art and include aldehydes, carbodiimides, and other bifunctional moieties.

The amount of osteogenic protein administered may vary depending upon the carrier used, the patient (age, sex, medical history, species) and the site and condition being treated. For local implantation, the weight ratio of osteogenic protein to carrier in the formulation will typically be in the range of about 1:5,000 to 1:50,000. The weight ratio of osteogenic protein to TGF-beta in the composition will usually be in the range of 10:1 to 1:10. The implant may be placed at a predetermined site in the patient by conventional surgical techniques, such as implantation or injection.

For systemic administration the amount of osteogenic protein will usually range between 30 g/kg body weight and 1 mg/kg body weight. TGF-beta may be added to the systemic formulations, if necessary, in the above proportions. In addition it may be desirable to combine the osteogenic protein with other therapeutics, such as, for instance in the case of osteoporosis, fluoride, calcitonin, vitamin D metabolites, and parathyroid hormone. Because the protein is nonspecies specific in its activity it may be used to treat mammals in general including sport, pet, and farm animals and humans.

Examples

The following is intended to illustrate the process for purification of native osteogenic protein as applied to a particular sample and the osteogenic activity of the isolated protein. It is not intended to limit the invention.

A. Preparation of Demineralized Bone

Bovine metatarsal bone was obtained fresh from the slaughterhouse and transported on ice. Bones were cleaned of all periosteum and marrow with high pressure water, crushed into fragments using a liquid-nitrogen-cooled grinder and pulverized into powder using a liquid-nitrogen-cooled mill. The pulverized bone was washed four times for 20 minutes in 4°C deionized water (8 liters/kg). The bone was then washed overnight with the same volume of deionized water at 4°C. The bone powder was demineralized for 5 hr in 0.5 N HCl (21 liter/kg) at 4°C. The acid was decanted, and the demineralized bone powder was washed several times with 4°C deionized water until the wash reached a pH > 3. The excess water was removed on a suction filter.

B. Extraction of Noncollagenous Proteins

Demineralized bone powder was extracted with 4 M guanidine-HCl, 10 mM EDTA pH 6.8 (2 liters/kg bone powder) for 16 hr at 4°C. The suspension was suction-filtered to recover the guanidine-HCl-soluble fraction and concentrated at least 5-fold by ultrafiltration using a 10,000 dalton cut-off membrane (S10Y10 Amicon spiral cartridge).

C. Gel Filtration

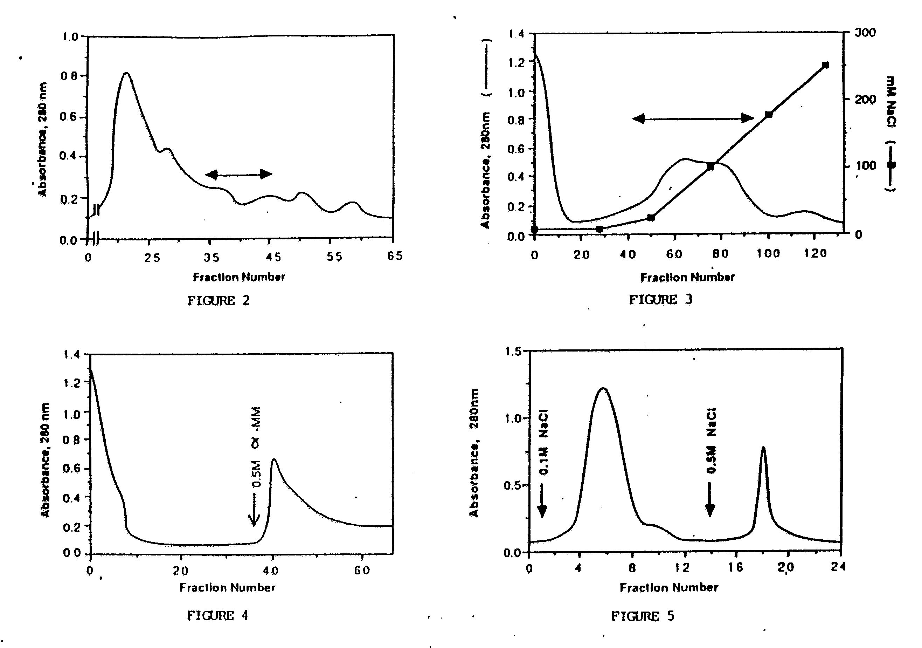

The extract from ©B, redissolved in 4 M guanidine-HCl, was fractionated on a Sephacryl S-200 column equilibrated in 4 M guanidine-HCl, 0.02% sodium azide, 10 mM EDTA, pH 6.8. Fractions were assayed by their absorbance at 280 nm and the fractions were combined as shown in Figure 2. The fraction indicated by < ----> in Figure 2 constitutes a low molecular weight (LMW, 10,000-30,000 daltons) protein fraction possessing the greatest activity. This fraction was pooled and dialyzed against 6 changes of 180 volumes of deionized water and lyophilized. All operations except lyophilization and dialysis (4°C) were conducted at room temperature.

D. Ion Exchange Chromatography

The pooled fraction from ©C was dissolved in 6 M urea, 10 mM NaCl, 1 mM NEM, 50 mM sodium acetate, pH 4.8 and centrifuged at 10,000 rpm for 5 min. The supernatant was fractionated on a CM52 (a commercially available CMC) column equilibrated in the same buffer. Bound proteins were eluted from the column using a 10 mM to 400 mM NaCl gradient in the same buffer, and a total volume of 350 ml at a flow rate of 27 ml/hr. Proteins eluted with 10-150 mM NaCl (the < ----> of Figure 3) were collected and dialyzed against 6 changes of 110 volumes of deionized water for 4 days and lyophilized. All of the foregoing operations were conducted at room temperature except dialysis (4°C).

E. ConA Chromatography

The fraction obtained in step D above was enriched in osteogenic activity by affinity chromatography using concanavalin A (ConA)-Sepharose 4B (Pharmacia). In order to minimize leaching of ConA from the column during chromatography, the resin was cross-linked with glutaraldehyde essentially as described by K.P. Campbell, D.H. MacLennan, J Biol Chem (1981) 256:4626. Briefly, resin was pelleted (500 x g, 5 min) and washed twice with 4 volumes of 250 mM NaHCO3, pH 8.8. The resin was then equilibrated in the same buffer for 6-8 hrs at 4°C. After pelleting, the resin was cross-linked by the addition of 4 volumes of 250 mM NaHCO3, pH 8.8, 250 mM methyl-alpha-D-mannopyranoside (alpha-MM), 0.03% glutaraldehyde with gentle mixing for 1 hr at room temperature. The reaction was quenched by washing the resin twice in 1 M Tris-HCl, pH 7.8. The resin was stored in the same buffer containing 0.01% Thimersol at 4°C until use.

Samples for ConA chromatography were solubilized in 1% deoxycholate at pH 8.0. Any small amount of precipitate was removed by centrifugation 12,000 x g, 5 minutes.

Prior to chromatography, cross-linked resin was first equilibrated with >5 column volumes of 50 mM Tris, pH 8.0 followed by >5 column volumes of 1% sodium deoxycholate. Samples were loaded and nonbound fractions collected by washing with 1% DOC. Elution was monitored by OD$_{280}$ Bound material was eluted with 0.5 M alpha-MM in 1% DOC as shown in Figure 4.

F. Chromatography on Heparin-Sepharose

The bound fraction eluted from the ConA column was reequilibrated by chromatography on a GH-25 column (Pharmacia) equilibrated in 6 M urea, 0.1 M NaCl, 50 mM Tris-HCl pH 7.2 heparin-sepharose buffer. Approximately 80 mg (1 mg/ml) were loaded on a 25 ml large heparin sepharose column (Pharmacia). The column was washed of all unbound material. Then bound proteins were eluted with the same equilibrating buffer but containing 0.5 M NaCl as shown in Figure 5. About 5-8 mg of heparin-sepharose bound proteins were recovered.

G. Chromatography on C18-RP-HPLC

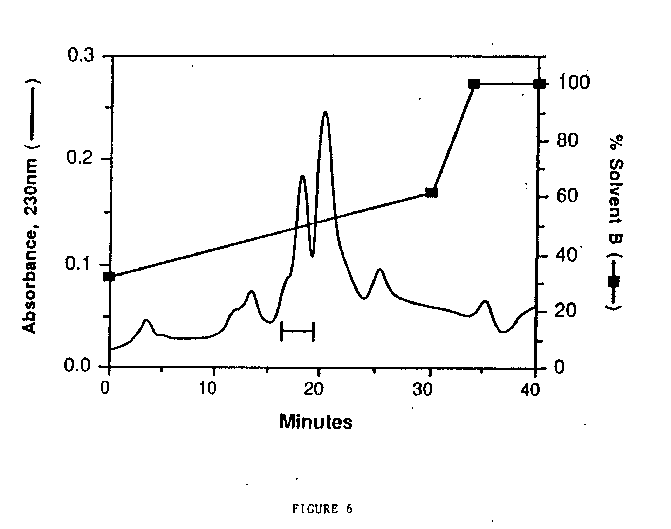

The pH of the heparin-bound fraction was lowered below 5 by adding TFA. Final purification of the heparin-bound fraction was achieved using reversed phase HPLC. The columns used were a Vydac TP-RP18 4.6 mm x 25 cm and 1.0 x 25 cm. Solvent A was 0.1% aqueous trifluoroacetic acid (TFA) and B 90% acetonitrile in A. Bound proteins were eluted from the column with a 32-62% B solvent gradient at a rate of 1%/min. The osteogenic protein composition eluted between 47-50% solvent B as shown in Figure 6. 140-200 ug protein were recovered. Amino acid composition and amino acid sequences of the protein were determined using standard procedures and are described above and shown in Figure 7.

H. Deglycosylation

Glycopeptidase F cleaves N-linked oligosaccharides at the innermost N-acetylglucosamine residue. High mannose, hybrid and complex oligosaccharides are susceptible to the enzyme. Osteogenic protein was iodinated by the chloramine-T method. Labeled protein was digested for 12-15 hours with 6.7 units/ml glycopeptidase F (Boehringer Mannheim) in 0.1 M Tris-HCl, pH 7.4, 10 mM EDTA, at 37°C.

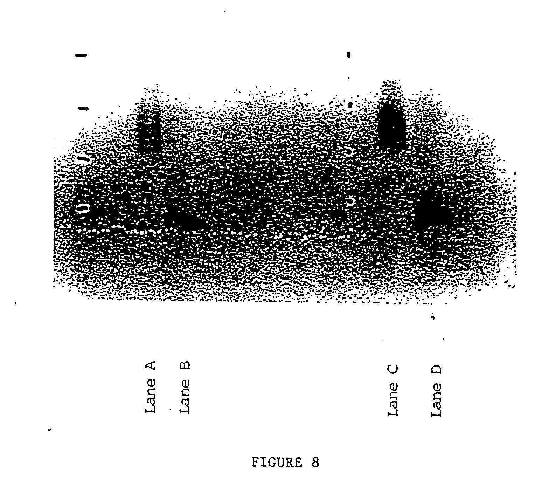

Both the glycosylated and deglycosylated forms were analyzed by sodium dodecyl sulfate/15% polyacrylamide slab gels prepared according to standard methods. Figure 8 is a photograph of the autoradiograph.

I. In vivo Bioassay of Materials

The osteoinductive activity of the protein composition described above was evaluated in vivo as follows.

I.1. Formulation of Osteogenic Protein Composition

The protein was used as a 24 ug/ml solution in 1% TFA, approximately 45% acetonitrile. TGF-beta2 was used as a 30 ug/ml solution in 1% TFA, approximately 45% acetonitrile.

One ml of osteogenic protein solution and 1.4 ml of the TGF-beta solution were stirred with 9 ml of Vitrogen® collagen-in-solution (Collagen Corporation, Palo Alto, CA) at 4°C for 5 min. 243 mg of porous hydroxylapatite/tricalciumphosphate ceramic in particulate form (Zimmer Corp., Warsaw, IN) was added and the mixture was incubated at 4°C for 5 min and then lyophilized. This mixture provided sufficient material for 6 implants. A like formulation was made lacking TGF-beta.

I.2. Implantation

The ability of the formulations of I.1 to induce endochondral bone formation in rats was determined as follows. Portions of the lyophilized formulations were [hydrated with approximately one volume of water, allowed to soak for 5 min and molded into approximately 5 x 5 mm bodies for implantation. The implants contained ~4 ug/implant of osteogenic protein and 0 or ~7 ug/implant of TGF-beta. The implants were surgically placed in 34-40 day old male Sprague-Dawley rats on either side of the ventral thoracic region. Explants were removed at 14 days and evaluated biochemically for bone formation.

I.3. Assay for Alkaline Phosphatase

The level of alkaline phosphatase (AP) activity in the explants is a measure of osteogenic activity. To determine alkaline phosphatase (AP), the explants were cut in small pieces and homogenized in 10 volumes (1/10) of ice-cold 1.5 M NaCl, 3 mM NaHCO3, pH 7.5 The homogenized samples were then centrifuged at 12,000 rpm for 50 min at 4°C, and an aliquot of the supernatant was diluted 1:10 in cold, distilled water. The method of Huggins, et al., J EXP Med (1961) 114:761, was used to assess alkaline phosphatase using polystyrene plates.

## I.4. Results

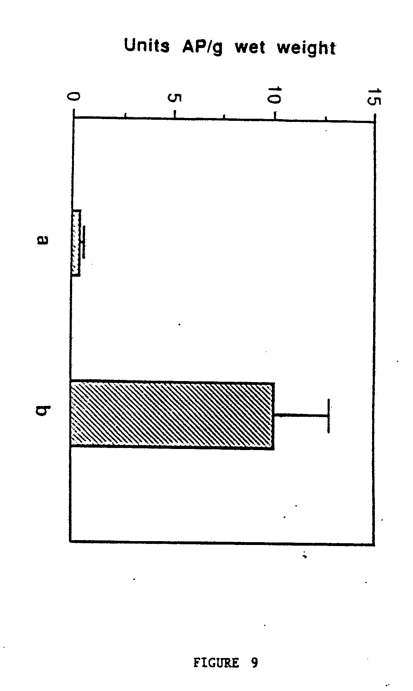

Figure 9 is a bar graph showing the results of the AP assay. As indicated the formulation containing no TGF-beta (a) was essentially non-active in the assay; whereas the formulation with TGF-beta (b) showed substantial activity. Histological examination of the explants confirmed the assay results.

Similar tests were carried out using osteogenic protein compositions that had been reduced and alkylated or trypsinized. These tests indicate that the protein is deactivated by such treatments.

## J. In vitro Assay of Materials

### J.1. Effect on Rat Osteosarcoma Cell Line (ROS 17/2.8)

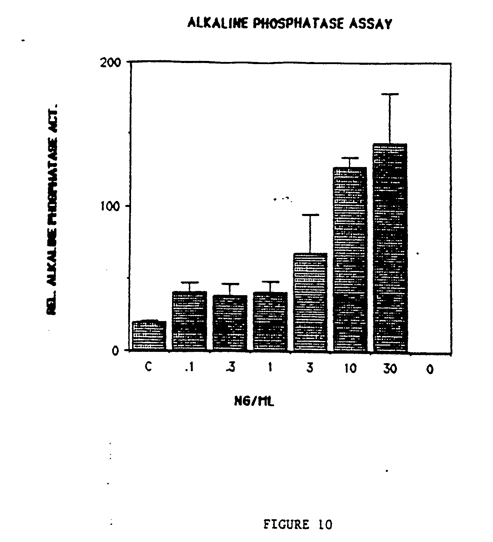

Cells were seeded in multiwell plates at 2500 cells/cm$^2$. After overnight attachment in Hams F12 medium, 10% fetal bovine serum, the cells were incubated with varying amounts of the osteogenic protein composition for 72 hours in serum-free Hams F12 medium. The control was serum-free Hams F12 medium containing no osteogenic protein. After incubation the cells were rinsed twice with PBS and lysed with 0.1% Triton. Aliquots were taken and tested for AP activity using p-nitrophenylphosphate (PNP) as a substrate. Figure 10 shows the results of those assays. As shown, the osteogenic protein produced a significant increase in AP activity which was dose-dependent at concentrations of 3-30 ng/ml.

### J.2. Effect on Adult Baboon Explant Bone Cells

Cancellous bone was obtained from adult baboon long bone by biopsy. The bone was minced and cellular outgrowth followed for up to three weeks. Cells were trypsined and used immediately or replated. Replated cells were used within the first three passages. Tests for AP activity were carried out as in J.1. above. The results of those tests were reported in the table below.

| Osteogenic Protein Concentration (ng/ml) | AP specific activity (nmPNP/ug protein/min) |
|---|---|
| 100 | 6.0 x 10$^{-3}$ |
| 50 | 9.1 x 10$^{-3}$ |
| 25 | 4.8 x 10$^{-3}$ |
| 12.5 | 3.5 x 10$^{-3}$ |
| Control | 3.0 x 10$^{-3}$ |

Similar tests were carried out on the human osteoblastic cell line MG-63 and on rat primary osteoblastic cells. However, no increase in AP activity was observed.

### J.3. Effect on Multinucleated Cell (MNC) Formation

Multinucleated cells are "osteoclast-like" cells. Osteoclasts are involved in bone resorption. Inhibition of the formation of osteoclasts is believed to limit bone resorption.

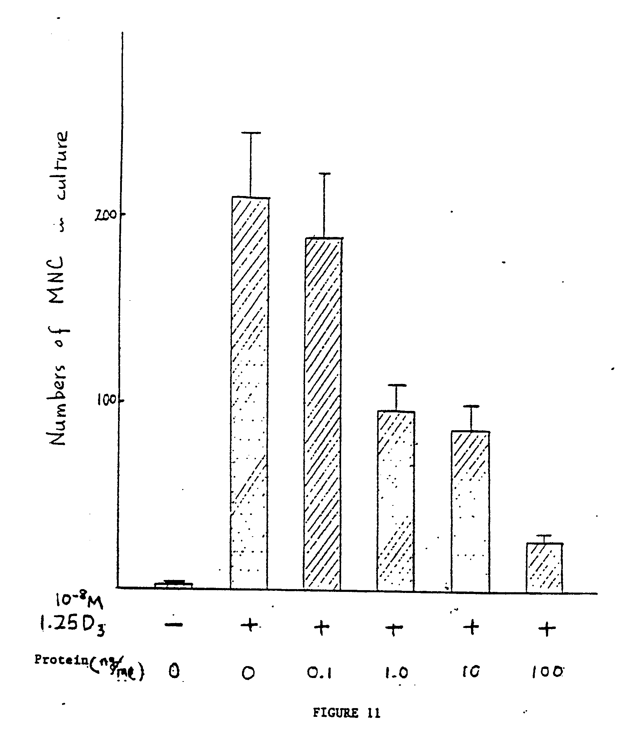

Bone marrow mononuclear cells from normal human donors were isolated using Hypaque-Ficoll (Histopaque-1077) gradient centrifugation. Mononuclear cells were cultured in alpha-MEM containing 20% horse serum at 10$^6$ cells/ml in 24-well plates (5x10$^5$ cells/well). All cultures were maintained in a humidified atmosphere of 4% CO$_2$-air at 37°C. Cultures were fed weekly by removing half of the medium and replacing an equal volume of fresh medium. Osteogenic protein was added to the cultures in the presence of 1,25-dihydroxyvitamin D (D$_3$), 10$^{-8}$M. After designated periods of culture (usually 3 weeks) cells were fixed with 5% glutaraldehyde and stained with Wright's stain. Cells containing 3 or more nuclei were counted as MNCs using an inverted phase microscope. Figure 11 is a graph showing the results of these tests. As shown, the osteogenic protein caused a dose-dependent inhibition of MNC formation at the concentrations tested.

## K. Production and Testing of Antibodies to the Osteogenic Protein

### K.1. Production of Polyclonal Antibodies

Polyclonal antibodies to (1) a synthetic 30 mer polypeptide having a sequence corresponding to the amino acids 1-30 of Figure 7 except for a Leu ----> Asn substitution at position 25 and (2) the native protein purified from bone as described above were prepared and characterized as follows.

Antiserum to the 1-30 mer was raised in a rabbit by injecting the rabbit with 500 ug of the polypeptide in complete Freund's adjuvant (CFA), followed by boosts of 500 ug of the polypeptide in incomplete Freund's adjuvant (ICFA) at approximately three week intervals. The antiserum was obtained after the fourth boost and had a titer as measured by ELISA of > 1:10,000. Rabbit antiserum to the native protein was raised similarly using an initial injection of 50 ug protein in CFA followed by boosts of 50 ug protein in ICFA. This antiserum had a titer of > 1:10,000 by ELISA.

The antiserum to the 1-30 mer was tested in Western blots on the purified native osteogenic protein, deglycosylated native osteogenic protein, and on crude native osteogenic protein (Con-A bound material), all fixed post-blotting with 0.2% glutaraldehyde. The antiserum detected the purified native osteogenic protein at ≧1 ug and also recognized the deglycosylated protein and the crude protein. The antiserum to the native osteogenic protein recognized the native protein at ≧100 ng in Western blots.

### K.2. Production of Monoclonal Antibodies

Murine monoclonal antibodies to the purified native osteogenic protein were prepared as follows. From two fusions 25 positive wells were identified. A group of female Balb/c mice was injected intraperitoneally (IP) with 10-20 ug of purified native osteogenic protein in CFA. The animals were boosted with 10-20 ug of protein in ICFA. Following the third boost, the mice were bled and serum antibody titers against the protein checked by ELISA. Two animals were found to have titers of ≧1:40,000. They were given a final intravenous (IV) injection of 20 ug protein four days prior to the fusion.

Fusion to the SP2/0 myeloma (GM3659 B, NIGMS Human Genetic Mutant Cell Repository, Camden, NJ) was performed essentially according to the protocol of Oi and Herzenberg, "Immunoglobulin-producing Hybrid Cell Lines" in Selected Methods in Cellular Immunology, Mishell and Shiigi, eds., W.H. Freeman and Co., San Francisco, pp. 357-362, (1980). Spleen cells from the animals were mixed with SP2/0 at a ratio of 5:1. 50% polyethylene glycol 1500 (Boehringer-Mannheim Biochemicals, Indianapolis, IN) was used as the fusagen. Cells were plated at $10^6$ cells/well along with resident peritoneal cells at $4 \times 10^3$ cells/well in DMEM with high glucose (4.5 g/l) supplemented with 20% FCS (Hyclone Laboratories, Logan, UT), 2 mM L-glutamine, 2 mM sodium pyruvate, nonessential amino acids, penicillin and streptomycin. In this procedure, aminopterin was replaced by azaserine (Sigma) according to the procedure by Larrick et al., Proc Natl Acad Sci USA (1983) 80:6376, and added along with thymidine and hypoxanthine on day 1 after the fusion.

From two fusions 25 positive wells were identified.

All 25 were positive for immunoprecipitation of $^{125}$I-labeled osteogenic protein and 23 of the 25 were positive in an ELISA against the protein. The supernatant from one uncloned well (3B2.17, previously designated FO13-3B2) was particularly positive and was used in a Western blot. In this testing synthetic peptides corresponding to amino acid segments 1-30, 62-95 and 76-105 of the osteogenic protein sequence were made and 1-2 ug of each was applied to separate lanes in the gel. Blots were probed with 50-100 ug/ml of purified antibody. This antibody recognized $\geq 300$ ng protein as well as deglycosylated protein. The antibody also picked up the protein in a crude fraction (total Con-A bound) and was found to recognize the C-terminal peptide (76-105) but not the N-terminal peptide (1-30). Another clone, designated 2C11.6, was found to recognize the internal 62-95 segment. Clones 3B2.17 and 2C11.6 were subcloned by limiting dilution and were found to be stable and to be IgG isotype. These clones are being deposited in the American Type Culture Collection (ATCC) under the provisions of the Budapest Treaty.

The antibodies produced by the two clones were testing for their ability to neutralize or block the activity of the osteogenic protein. ROS or BMS-2 cells were cultured with 30 ng/ml of the osteogenic protein in the presence or absence of antibody at varying concentrations. Both of these cell lines normally exhibit substantial increases in AP when cultured in media containing osteogenic protein at 30 ng/ml. The antibody from 3B2.17 was found to neutralize this effect of the osteogenic protein at concentrations >10 ug/ml.

Modifications of the above-described modes of carrying out the invention that are obvious to those of skill in the arts relevant to the invention are intended to be within the scope of the following claims.

## Claims

1. A substantially pure polypeptide having osteogenic activity and an internal sequence in the N-terminal portion as follows
-Lys-Tyr-Asn-Lys-Ile-Lys-Ser-Arg-Gly-Ile-Lys-Ala-Asn-Thr-Phe-Lys-Lys-Leu-His-Asn-Leu-Ser-Phe-Leu-Tyr-Leu-Asp-His-Asn-Ala-Leu-Glu-
and substantially pure polypeptides that are substantially equivalent and substantially homologous thereto.

2. The polypeptide of claim 1 wherein the amino acid immediately preceding the initial Lys of the internal sequence is Ala and defines the amino terminal of the polypeptide.

3. A polypeptide having an internal sequence in the N-terminal portion of the polypeptide as follows:
-Lys-Tyr-Asn-Lys-Ile-Lys-Ser-Arg-Gly-Ile-Lys-Ala-Asn-Thr-Phe-Lys-Lys-Leu-His-Asn-Leu-Ser-Phe-Leu-Tyr-Leu-Asp-His-Asn-Ala-Leu-Glu-
wherein said polypeptide is deglycosylated relative to a native osteogenically active polypeptide having said sequence and deglycosylated polypeptides that are substantially equivalent and substantially homologous thereto.

4. A substantially pure osteogenically active polypeptide having the following amino acid sequence:
($H_2N$)-Ala-Lys-Tyr-Asn-Lys-Ile-Lys-Ser-Arg-Gly-Ile-Lys-Ala-Asn-Thr-Phe-Lys-Lys-Leu-His-Asn-Leu-Ser-Phe-Leu-Tyr-Leu-Asp-His-Asn-Ala-Leu-Glu-Ser-Val-Pro-Leu-Asn-Leu-Pro-Glu-Ser-Leu-Arg-Val-Ile-His-Leu-Gln-Phe-Asn-Asn-Ile-Thr-Ser-Ile-Thr-As p-Asp-Thr-Phe-Cys-Lys-Ala-Asn-Asp-Thr-Ser-Tyr-Ile-Arg-Asp-Arg-Ile-Glu-Glu-Ile-Arg-Leu-Glu-Gly-Asn-Pro-Val-Ile-Leu-Gly-Lys-His-Pro-Asn-Ser-Phe-Ile-Cys-Leu-Lys-Arg-Leu-Pro-Ile-Gly-Ser-Tyr-Ile-Asp-(COOH),
and substantially pure polypeptides that are substantially equivalent and substantially homologous thereto.

5. A polypeptide having the following amino acid sequence:
($H_2N$)-Ala-Lys-Tyr-Asn-Lys-Ile-Lys-Ser-Arg-Gly-Ile-Lys-Ala-Asn-Thr-Phe-Lys-Lys-Leu-His-Asn-Leu-Ser-Phe-Leu-Tyr-Leu-Asp-His-Asn-Ala-Leu-Glu-Ser-Val-Pro-Leu-Asn-Leu-Pro-Glu-Ser-Leu-Arg-Val-Ile-His-Leu-Gln-Phe-Asn-Asn-Ile-Thr-Ser-Ile-Thr-As p-Asp-Thr-Phe-Cys-Lys-Ala-Asn-Asp-Thr-Ser-Tyr-Ile-Arg-Asp-Arg-Ile-Glu-Glu-Ile-Arg-Leu-Glu-Gly-Asn-Pro-Val-Ile-Leu-Gly-Lys-His-Pro-Asn-Ser-Phe-Ile-Cys-Leu-Lys-Arg-Leu-Pro-Ile-Gly-Ser-Tyr-Ile-Asp-(COOH),
wherein said polypeptide is deglycosylated relative to a native osteogenically active polypeptide having said sequence and deglycosylated polypeptides that are substantially equivalent and substantially homologous thereto.

6. A composition for inducing bone formation and/or inhibiting bone resorption comprising an effective amount of an osteogenically active polypeptide of claim 1, 2 or 4.

7. The composition of claim 6 which further includes an effective amount of TGF-beta.

8. A method of inducing bone formation in vivo at a predetermined site in a living mammal comprising placing the composition of claim 6 or 7 at said site.

9. A method of inducing bone marrow cell production in a living mammal comprising administering an effective amount of the composition of claim 6 or 7 to the mammal systemically.

10. A method of treating osteoporosis in a living mammal comprising administering an effective amount of the composition of claim 6 or 7 to the mammal systemically.

11. In the method of treating a living mammal for a cancer of the hematopoietic system comprising irradiating the mammal to kill neoplastic hematopoietic cells, the improvement comprising administering a sufficient amount of the composition of claim 6 or 7 to the mammal systemically after said irradiation to stimulate hematopoietic stem cell division.

12. The method of claim 11 wherein a sufficient amount of TGF-beta is administered systemically to the mammal prior to said irradiation to suppress hematopoietic stem cell division.

13. Antibody that binds to a polypeptide of claim 1, 2, 3, 4 or 5.

14. A recombinant polynucleotide encoding a polypeptide of claim 1, 2, 3, 4 or 5.

15. A recombinant vector containing a recombinant polynucleotide of claim 14 and capable of directing the expression of the polypeptide encoded thereby.

16. A recombinant host cell or microorganism containing the recombinant vector of claim 15 and capable of permitting expression of said polypeptide.

17. A process for producing a polypeptide of claim 1, 2, 3, 4 or 5 comprising culturing the recombinant host cell or microorganism of claim 16.

DEMINERALIZED BONE POWDER ( 0.5 N HCL )

↓

4 M GUANIDINE HYDROCHLORIDE EXTRACT

↓

SEPHACRYL S - 200 ( 20 - 36KD MW )

↓

CM - CELLULOSE ( 10 - 150 mM NaCL )

↓

CONCANAVALIN - A ( 0.5 M Methyl α- D - Mannopyranoside )

↓

HEPARIN - SEPHAROSE ( 0.1 - 0.5 M NaCL )

↓

C 18 - REVERSED PHASE - HPLC ( 42 - 45% Acetonitrile )

FIGURE 1

FIGURE 2

FIGURE 3

FIGURE 4

FIGURE 5

FIGURE 6

EP 0 336 760 A2

| AA # | N-TERMINUS | LYS-C | GLU-C | LYS-C | GLU-C | LYS-C |
|---|---|---|---|---|---|---|
| 1 | ALA | | | | | |
| 2 | LYS | | | | | |
| 3 | TYR | | | | | |
| 4 | ASN | | | | | |
| 5 | LYS | | | | | |
| 6 | ILE | | | | | |
| 7 | LYS | | | | | |
| 8 | SER | | | | | |
| 9 | ARG | | | | | |
| 10 | GLY | | | | | |
| 11 | ILE | | | | | |
| 12 | LYS | | | | | |
| 13 | ALA | | | | | |
| 14 | ASN | | | | | |
| 15 | THR | | | | | |
| 16 | PHE | | | | | |
| 17 | LYS | | | | | |
| 18 | LYS | (LYS) | | | | |
| 19 | LEU | LEU | | | | |
| 20 | HIS | HIS | | | | |
| 21 | ASN | ASN | | | | |
| 22 | LEU | LEU | | | | |
| 23 | SER | SER | | | | |
| 24 | PHE | PHE | | | | |
| 25 | LEU | LEU | | | | |
| 26 | TYR | TYR | | | | |
| 27 | LEU | LEU | | | | |
| 28 | ASP | ASP | | | | |
| 29 | HIS | HIS | | | | |
| 30 | ASN | ASN | | | | |
| 31 | ALA | ALA | | | | |
| 32 | LEU | LEU | | | | |
| 33 | GLU | GLU | | | | |
| 34 | | SER | | | | |
| 35 | | VAL | | | | |
| 36 | | PRO | | | | |
| 37 | | LEU | | | | |
| 38 | | ASN | | | | |
| 39 | | LEU | | | | |
| 40 | | PRO | | | | |
| 41 | | GLU | | | | |
| 42 | | SER | SER | | | |
| 43 | | LEU | LEU | | | |
| 44 | | | ARG | | | |
| 45 | | | VAL | | | |
| 46 | | | ILE | | | |
| 47 | | | HIS | | | |
| 48 | | | LEU | | | |
| 49 | | | GLN | | | |
| 50 | | | PHE | | | |
| 51 | | | ASN | | | |
| 52 | | | ASN | | | |
| 53 | | | ILE | | | |
| 54 | | | THR | | | |
| 55 | | | SER | | | |
| 56 | | | ILE | | | |
| 57 | | | THR | | | |
| 58 | | | ASP | | | |
| 59 | | | ASP | | | |
| 60 | | | THR | | | |
| 61 | | | PHE | | | |
| 62 | | | CYS | | | |
| 63 | | | LYS | | | |
| 64 | | | ALA | ALA | | |
| 65 | | | | ASN-CHO | | |
| 66 | | | | ASP | | |
| 67 | | | | THR | | |
| 68 | | | | SER | | |
| 69 | | | | TYR | | |
| 70 | | | | ILE | | |
| 71 | | | | ARG | | |
| 72 | | | | ASP | | |
| 73 | | | | ARG | | |
| 74 | | | | ILE | | |
| 75 | | | | GLU | | |
| 76 | | | | GLU | | |
| 77 | | | | ILE | | |
| 78 | | | | ARG | | |
| 79 | | | | LEU | | |
| 80 | | | | GLU | | |
| 81 | | | | GLY | GLY | |
| 82 | | | | ASN | ASN | |
| 83 | | | | PRO | PRO | |
| 84 | | | | VAL | VAL | |
| 85 | | | | ILE | ILE | |
| 86 | | | | LEU | LEU | |
| 87 | | | | GLY | GLY | |
| 88 | | | | LYS | LYS | |
| 89 | | | | | HIS | |
| 90 | | | | | PRO | |
| 91 | | | | | ASN | |
| 92 | | | | | SER | |
| 93 | | | | | PHE | |
| 94 | | | | | ILE | |
| 95 | | | | | CYS | |
| 96 | | | | | LEU | |
| 97 | | | | | LYS | |
| 98 | | | | | ARG | ARG |
| 99 | | | | | LEU | LEU |
| 100 | | | | | PRO | PRO |
| 101 | | | | | ILE | ILE |
| 102 | | | | | GLY | GLY |
| 103 | | | | | SER | SER |
| 104 | | | | | TYR | TYR |
| 105 | | | | | | ILE |
| 106 | | | | | | ASP-COOH |

FIGURE 7

Lane A
Lane B

Lane C
Lane D

FIGURE 8

FIGURE 9

ALKALINE PHOSPHATASE ASSAY

FIGURE 10

FIGURE 11